(19) Europäisches Patentamt

European Patent Office

Office européen des brevets

(11) **EP 3 825 872 B1**

(12) # EUROPEAN PATENT SPECIFICATION

(45) Date of publication and mention
of the grant of the patent:
**10.08.2022 Bulletin 2022/32**

(21) Application number: **20208447.1**

(22) Date of filing: **18.11.2020**

(51) International Patent Classification (IPC):
**G06F 16/901** *(2019.01)* **G16B 5/00** *(2019.01)*
**G16C 20/10** *(2019.01)*

(52) Cooperative Patent Classification (CPC):
**G06F 16/9024; G16B 5/00; G16C 20/10;
G16C 20/70**

(54) **INFORMATION PROCESSING SYSTEM AND SEARCH METHOD**

INFORMATIONSVERARBEITUNGSSYSTEM UND SUCHVERFAHREN

SYSTÈME DE TRAITEMENT D'INFORMATIONS ET PROCÉDÉ DE RECHERCHE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.11.2019 JP 2019210138**

(43) Date of publication of application:
**26.05.2021 Bulletin 2021/21**

(73) Proprietor: **Hitachi, Ltd.
Tokyo 100-8280 (JP)**

(72) Inventors:
• **KATO, Masahiro**
**Tokyo, 100-8280 (JP)**
• **ITO, Kiyoto**
**Tokyo, 100-8280 (JP)**
• **IMAICHI, Osamu**
**Tokyo, 100-8280 (JP)**

(74) Representative: **MERH-IP Matias Erny Reichl
Hoffmann
Patentanwälte PartG mbB
Paul-Heyse-Strasse 29
80336 München (DE)**

(56) References cited:
**US-A1- 2018 101 663**

• **PARTL CHRISTIAN ET AL: "ConTour:
Data-Driven Exploration of Multi-Relational
Datasets for Drug Discovery", IEEE
TRANSACTIONS ON VISUALIZATION AND
COMPUTER GRAPHICS, IEEE SERVICE
CENTER, LOS ALAMITOS, CA, US, vol. 20, no. 12,
31 December 2014 (2014-12-31), pages
1883-1892, XP011563339, ISSN: 1077-2626, DOI:
10.1109/TVCG.2014.2346752 [retrieved on
2014-10-23]**
• **OVACIK MERIC A ET AL: "Enzyme sequence
similarity improves the reaction alignment
method for cross-species pathway comparison",
TOXICOLOGY AND APPLIED PHARMACOLOGY,
vol. 271, no. 3, 17 September 2010 (2010-09-17),
pages 363-371, XP028711621, ISSN: 0041-008X,
DOI: 10.1016/J.TAAP.2010.09.009**
• **LI YUNLEI ET AL: "Metabolic pathway alignment
between species using a comprehensive and
flexible similarity measure", BMC SYSTEMS
BIOLOGY, BIOMED CENTRAL LTD, LO, vol. 2, no.
1, 24 December 2008 (2008-12-24), page 111,
XP021052418, ISSN: 1752-0509, DOI:
10.1186/1752-0509-2-111**

**EP 3 825 872 B1**

**Description**

BACKGROUND OF THE INVENTION

1. Field of the Invention

The present invention relates to an information processing system and a search method for searching a literature.

2. Description of the Related Art

In recent years, it has become common to search a literature database for literatures related to a subject to be solved, find useful literatures, and use the literatures for research.

There is a technique related to a search system, in which literatures having a high similarity to a search expression or a high correlation coefficient are extracted, an extended search expression is created by selecting words based on the extracted literatures, and the extended search expression is used to perform searching. For example, JP-A-2002-215672  describes such a technique.

PARTL CHRISTIAN ET AL: "ConTour: Data-Driven Exploration of Multi-Relational Datasets for Drug Discovery", IEEE TRANSACTIONS ON VISUALIZATION AND COMPUTER GRAPHICS, IEEE SERVICE CENTER, LOS ALAMITOS, CA, US, vol. 20, no. 12, 31 December 2014 (2014-12-31), pages 1883-1892, XP011563339, ISSN: 1077-2626, DOI: 10.1109/TVCG.2014.2346752 [retrieved on 2014-10-23], describes a graph for visualization of metabolic pathways.

SUMMARY OF THE INVENTION

The invention is defined by the features of the independent claims. Further embodiments are the subject-matter of the dependent claims.

An inventor of the invention has found that, in a literature search related to natural science, for example, a field of biochemistry, literatures that a user wants to extract may be more suitably extracted by performing a search based on relationships (natural laws) of a physical world in the field to be searched.

Therefore, the invention provides an information processing system and a search method which are suitable for searching for a literature related to metabolism for synthesizing substances.

A configuration of an information processing system of the invention is an information processing system that searches a literature database for a literature, and the information processing system includes: a database including pathway information related to a metabolic pathway that at least includes information on, for each of a plurality of metabolic reactions, a compound produced by reaction, a compound used in reaction, and enzymes that act in the metabolic reaction; an input unit configured to receive an input of information on a first reaction compound which was produced by reaction, a first reaction compound which was used in reaction, and a first enzyme that acts in the metabolic reaction for literature search, as pathway information of metabolic reaction for literature search; an extraction unit configured to extract, based on the information on the first reaction compound which was produced by reaction, the first reaction compound which was used in reaction, and the first enzyme, pathway information that has a predetermined relationship with the pathway information of metabolic reaction for literature search from the database, and extract, from the extracted pathway information, information on a peripheral compound that is different from the first reaction compound which was produced by reaction and the first reaction compound which was used in reaction; and a search unit configured to search the literature database for a literature based on the pathway information of metabolic reaction for literature search and the information on the peripheral compound.

According to the invention, it is possible to provide an information processing system and a search method which are suitable for searching for a literature related to metabolism for synthesizing substances.

Problems, configurations, and effects other than those described above will be apparent with reference to the description of following embodiments.

BRIEF DESCRIPTION OF THE DRAWINGS

FIG. 1 is a system configuration diagram of a literature search system.
FIG. 2 is a hardware configuration diagram of a literature search device.
FIG. 3 is a software configuration diagram of the literature search device.
FIG. 4 is a diagram showing an example of an input metabolic pathway.
FIG. 5 is a diagram showing an example of a metabolic map.

FIG. 6 is a diagram showing an example of a gene list.

FIG. 7 is a diagram showing an example of a literature set list.

FIG. 8 is a diagram showing an example of a search expression.

FIG. 9 is a diagram showing an example of a search result table.

FIG. 10 is a flowchart showing overall processing of the literature search device from inputting an input metabolic pathway to outputting a literature search result.

FIG. 11 is a flowchart showing peripheral compound information generation processing.

FIG. 12 is a flowchart showing metabolic map selection processing.

FIG. 13 is an example schematically showing an input metabolic pathway and peripheral compounds.

FIG. 14 is a diagram showing an adjacency matrix of the metabolic map of FIG. 13.

FIGS. 15A to 15D shows 3x3 partial adjacency matrices created based on the adjacency matrix of the metabolic map of FIG. 14.

FIG. 16 is a diagram showing a distance matrix of the metabolic map of FIG. 13.

FIG. 17 is a flowchart showing synonymous expression generation processing.

FIG. 18 is a diagram showing an example of labeling a compound with a similarity to a substrate.

FIG. 19 is a diagram showing similarity calculation.

FIG. 20 is a diagram showing literature score calculation processing.

FIG. 21 is a diagram showing an example of a search information output screen.

FIG. 22 is a diagram showing an example of a search result output screen.

## DESCRIPTION OF THE PREFERRED EMBODIMENTS

Hereinafter, embodiments of the present invention will be described with reference to the drawings. The following description and drawings are examples for describing the invention, and are omitted and simplified as appropriate for clarification of the description. The invention can be implemented in various other forms. Unless otherwise limited, each component may be singular or plural.

In order to facilitate understanding of the invention, a position, a size, a shape, a range, or the like of each configuration shown in the drawings may not represent an actual position, size, shape, range, or the like. Therefore, the present invention is not necessarily limited to the position, size, shape, range, or the like disclosed in the drawings.

In the following description, although various types of information may be described in terms of expressions such as "table", "list" and "queue", the various types of information may be expressed by other data structures. An "XX table", an "XX list", and the like are referred to as "XX information" to indicate that information does not depend on a data structure. When identification information is described, expressions such as "identification information", "identifier", "name", "ID", and "number" are used, but these expressions may be replaced with one another.

When there are a plurality of constituent elements having the same or similar function, different subscripts may be attached to the same reference numeral. However, when there is no need to distinguish the plurality of constituent elements, the subscripts may be omitted.

In the following description, processing performed by executing a program may be described. The program is executed by a processor (for example, a central processing unit (CPU) or a graphics processing unit (GPU)) appropriately performing a predetermined processing using a storage resource (for example, a memory) and/or an interface device (for example, a communication port), or the like. Therefore, the processor may serve as a subject of the processing. Similarly, the subject of the processing performed by executing the program may be a controller, device, system, computer, or node including a processor therein. The subject of the processing performed by executing the program may be a calculation unit, and may include a dedicated circuit (for example, an FPGA or an ASIC) for performing a specific processing.

The program may be installed from a program source into a device such as a computer. The program source may be, for example, a program distribution server or a computer-readable storage medium. When the program source is the program distribution server, the program distribution server may include a processor and a storage resource that stores a program to be distributed, and the processor of the program distribution server may distribute the program to be distributed to another computer. Two or more programs may be implemented as one program, or one program may be implemented as two or more programs in the following description.

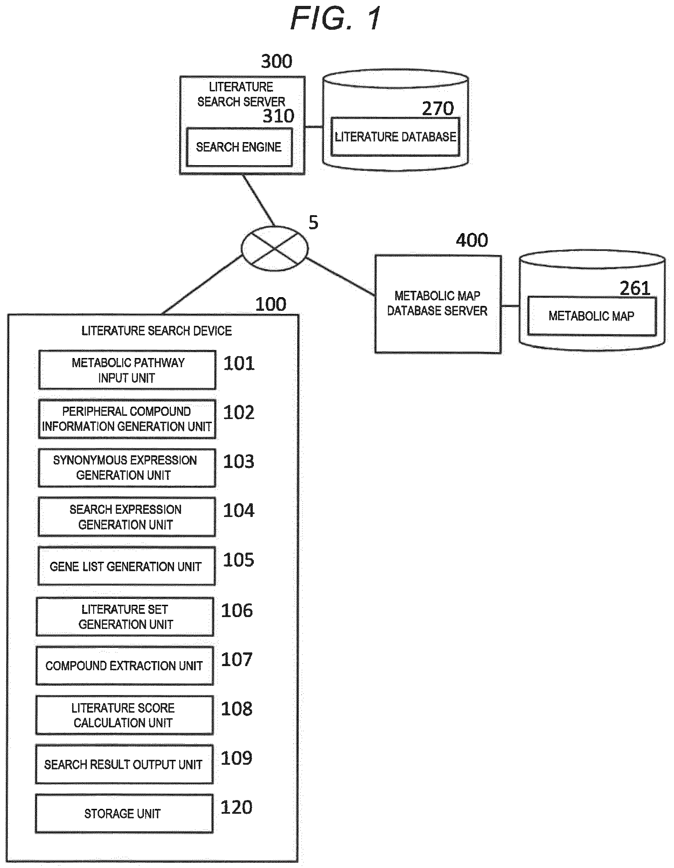

An embodiment according to the invention will be described below with reference to FIG. 1 to FIG. 22.

The present embodiment relates to a literature search system in which in order to design a microorganism (smart cell) that produces a target substance by genetic recombination, literature information on enzymes, genes, and microorganisms related to a metabolic pathway (compound and a chain that reacts with the same) in a living body can be more efficiently obtained by inputting the metabolic pathway. The literature search system is an information processing system.

As shown in FIG. 1, the literature search system has a configuration in which a literature search device 100, a literature search server 300, and a metabolic map database server 400 are connected by a network 5. The network 5 may be a local area network (LAN) or a global network such as an internet.

The literature search server 300 is a server device that receives a search expression from a client connected to the network, searches a literature database 270 for corresponding literature information, and returns the information to the client. The literature search server 300 includes a search engine 310. The search engine 310 is a functional unit that searches the literature database 270 for the corresponding literature information by inputting the search expression. The literature search server 300 described in the present embodiment will be described by taking PubMed as an example. The PubMed is a search engine for MEDLINE, on which references and abstracts related to life sciences and biomedical sciences are published.

The metabolic map database server 400 is a database server for a metabolic map 261. In the present embodiment, a Kyoto Encyclopedia of Genes and Genomes (KEGG) metabolic map database (KEGG PATHWAY Database) will be described as an example.

The literature search device 100, as shown in FIG. 1, has a functional configuration including a metabolic pathway input unit 101, a peripheral compound information generation unit 102, a synonymous expression generation unit 103, a search expression generation unit 104, a gene list generation unit 105, a literature set generation unit 106, a compound extraction unit 107, a literature score calculation unit 108, a search result output unit 109, and a storage unit 120.

The metabolic pathway input unit 101 is a functional unit with which a metabolic pathway (hereinafter referred to as "input metabolic pathway") is input as an input of a search condition. The metabolic pathway of the present embodiment is described by a directed graph in which a substrate (a reaction compound in a metabolic reaction) is set as a node, and an enzyme that acts when a node of a substrate used in reaction is moved to a node of a substrate produced by reaction is set as an edge. A specific sequence of the input metabolic pathway will be described in detail later.

The peripheral compound information generation unit 102 is a functional unit that generates information on compounds (hereinafter referred to as "peripheral compounds", and details will be described later) that exist near a graph of the input metabolic pathway by referring to a metabolic map (details will be described later) expressing metabolism.

The synonymous expression generation unit 103 is a functional unit that generates information on a compound that is synonymous with a peripheral compound (such as a compound that has the same expression but has a different name due to convention). The search expression generation unit 104 is a functional unit that generates a search expression (query) for literature search based on the information on the input metabolic pathway, the peripheral compounds, and the information on the compounds having synonymous expressions. The gene list generation unit 105 is a functional unit that generates a list showing a related gene and microorganism for each enzyme. The literature set generation unit 106 is a functional unit that generates a list (literature set list) of titles and abstracts of respective literatures. The compound extraction unit 107 is a functional unit that extracts a related compound from the literature set list. The literature score calculation unit 108 is a functional unit that calculates a literature score (details will be described later) obtained based on the similarity between the substrate and the compound. The search result output unit 109 is a functional unit that transmits the search expression to the literature search server 300 and outputs a search result from the literature search server 300.

The storage unit 120 is a functional unit that stores data necessary for the literature search device 100.

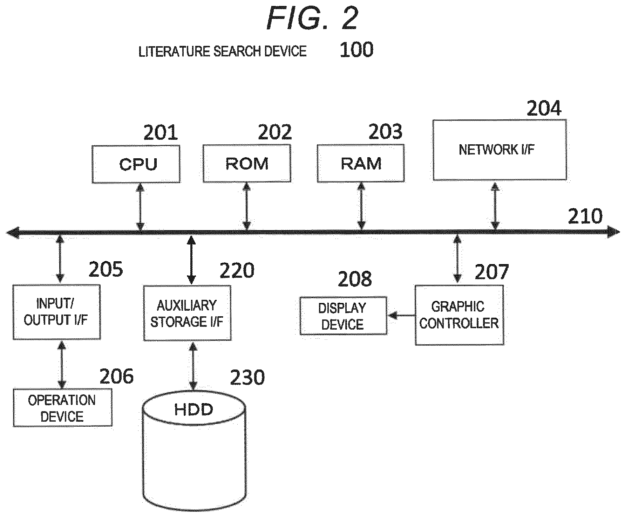

Next, a hardware configuration of the literature search device will be described with reference to FIG. 2.

The literature search device 100 of the present embodiment can be implemented by a general information processing device as shown in FIG. 2, and is connected to a display device 208 capable of displaying video.

The literature search device 100 has a hardware configuration in which a central processing unit (CPU) 201, a read only memory (ROM) 202, a random access memory (RAM) 203, a network interface (I/F) 204, an input/output I/F 205, a graphic controller 207, and an auxiliary storage I/F 220 are connected via an internal bus line 210.

The CPU 201 functions as a control unit that executes information processing, and implements various processes by executing a program stored in the ROM 202 or the RAM 203. The RAM 203 is a volatile semiconductor memory, and holds programs and work data loaded from an auxiliary storage device. The ROM 202 is a non-volatile semiconductor memory, and stores a basic program such as BIOS.

The network interface 204 is an interface unit for connecting to the network 5. For example, an analog modem for analog telephone lines, a modem for ISDN lines, a router or modem for asymmetric digital subscriber line (ADSL), an adapter for wireless communication, such as an adapter for local area network (LAN), or an adapter for wireless phone, or a Bluetooth (registered trademark), and the like are applicable. The literature search device 100 can be connected to the Internet via the interfaces of these configurations.

The graphic controller 207 is a controller for connecting the display device 208 and controlling display of various information and moving images. As the display device 208, for example, a liquid crystal display panel or the like is used.

The input/output I/F is an I/F connected to an operation device 206 such as a keyboard and a mouse.

The auxiliary storage device 220 is an interface for connecting to the auxiliary storage device such as a hard disk drive (HDD) 230. The auxiliary storage device may be a solid state drive SDD (SDD), or a removable storage device such as a memory card.

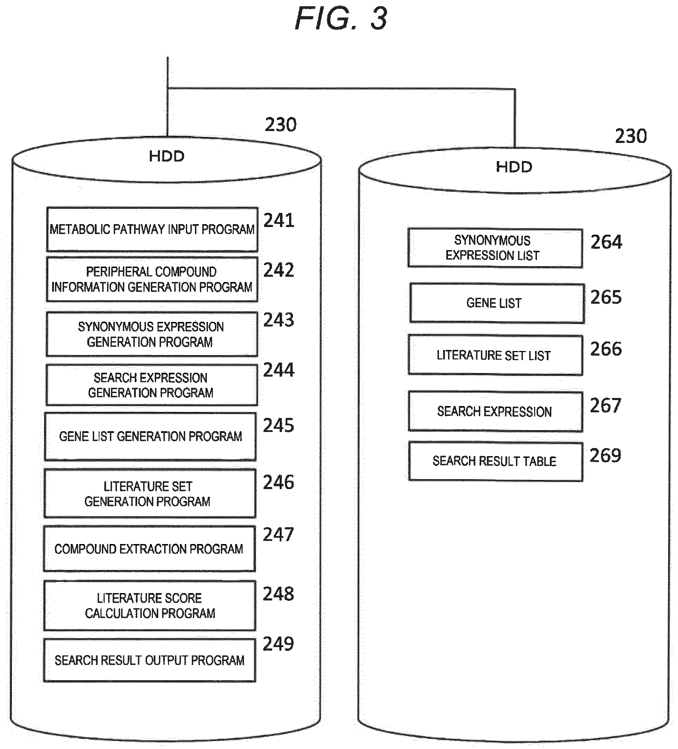

Next, a software configuration of the literature search device will be described with reference to FIG. 3.

The HDD 230 is a large-capacity storage device, and as shown in Fig. 3, a metabolic pathway input program 241, a peripheral compound information generation program 242, a synonymous expression generation program 243, a search expression generation program 244, a gene list generation program 245, a literature set generation program 246, a compound extraction program 247, a literature score calculation program 248, and a search result output program 249 are installed in the HDD 230.

The metabolic pathway input program 241, the peripheral compound information generation program 242, the synonymous expression generation program 243, the search expression generation program 244, the gene list generation program 245, the literature set generation program 246, the compound extraction program 247, the literature score calculation program 248, and the search result output program 249 are programs that can respectively realize functions of the metabolic pathway input unit 101, the peripheral compound information generation unit 102, the synonymous expression generation unit 103, the search expression generation unit 104, the gene list generation unit 105, the literature set generation unit 106, the compound extraction unit 107, the literature score calculation unit 108, and the search result output unit 109.

Further, the HDD 230 stores a synonymous expression list 264, a gene list 265, a literature set list 266, a search expression 267, and a search result table 269. A data structure used in the literature search system will be described in detail below.

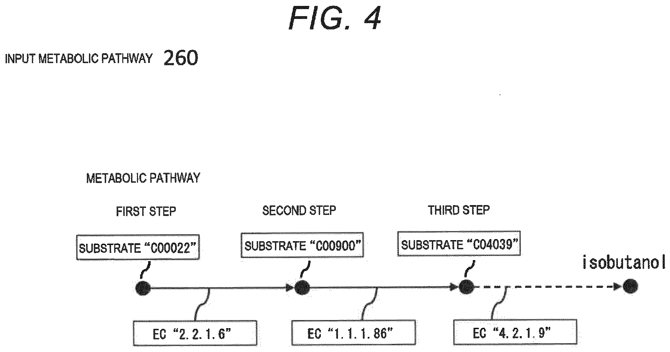

Next, the data structure used in the literature search system according to the present embodiment will be described with reference to FIGS. 4 to 9.

An input metabolic pathway 260 indicates a metabolic pathway in a living body that is input for searching, and as shown in FIG. 4, is described by a directed graph in which a substrate (a reaction compound in a metabolic reaction) is set as a node, and an enzyme that acts when a node of a substrate used in reaction is moved to a node of a substrate produced by reaction is set as an edge. This example shows a metabolic pathway that finally produces isobutanol through metabolic pathways referred to as a first step, a second step, a third step, and the like. The node is a reaction compound, that is a substrate, and the edge that connects the nodes is an enzyme that reacts specifically with a base substrate. A reaction with the enzyme produces a substrate at a tip end of an arrow, and a compound produced in this reaction is used again as a substrate for a next step, thus forming a reaction chain. The substrate and enzyme at each step are respectively described by a compound ID and an EC number (Enzyme Commission numbers) . Here, the EC number is an identifier defined by Enzyme Committee of International Union of Biochemistry (now the International Union of Biochemistry and Molecular Biology (IUBMB)) and is represented by 4 numbers as a set following EC according to a reaction format for organizing enzymes. The example shown in FIG. 4 shows that a substrate in the first step is described as "C00022" and the enzyme is "EC 2.2.1.6", and a reaction with the enzyme produces a substrate "C00900" in the second step.

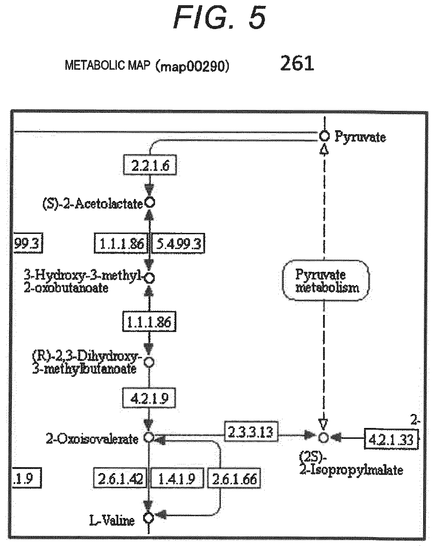

The metabolic map 261 is a diagrammatic representation of metabolic pathways (Pathway) in a living body or a network including the pathways, and is described by a directed graph in which a substrate (a reaction compound in a metabolic reaction) is set as a node, and an enzyme that acts when a node of a substrate used in reaction is moved to a node of a substrate produced by reaction is set as an edge. In the present embodiment, as described above, the metabolic map provided by the KEGG metabolic map database will be described as an example . The example shown in FIG. 5 shows that by selecting one of the metabolic maps (map00290) linked to an item referred to as Pathway on a page that is searched in the KEGG metabolic map database with an enzyme "EC 4.2.1.9" in the third step of the input metabolic pathway in FIG. 4, a periphery of the enzyme "EC 4.2.1.9" in the third step is cut off.

The synonymous expression list 264 is a list of compounds that are labeled depending on the same similarity.

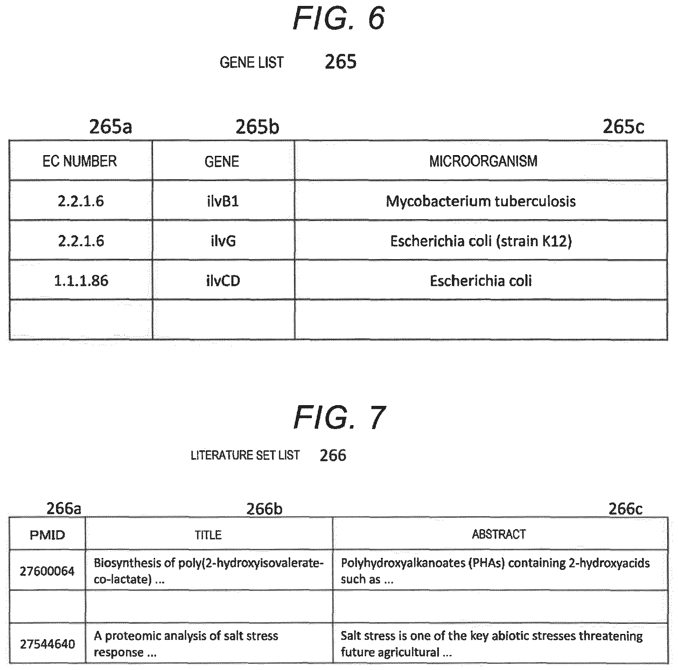

The gene list 265 is a table showing genes and microorganisms related to enzymes of the input metabolic pathway, and as shown in FIG. 6, includes items of an EC number 265a, a gene 265b, and a microorganism 265c. The item of EC number 265a stores the EC numbers of the enzymes involved in the input metabolic pathway. The item of gene 265b stores a symbol representing a gene that expresses the enzyme . The item of microorganism 265c stores a microorganism (bacteria, and the like) serving as a host of a gene. In the example of FIG. 6, ilvB1 is a gene corresponding to the enzyme whose EC number is 2.2.1.6, and Mycobacterium tuberculosis is an organism serving as a host of the ilvB1.

The literature set list 266 is a list that stores information on titles and abstracts for literatures, and as shown in FIG. 7, includes items of a literature ID 266a, a title 266b, and an abstract 266c. The item of literature ID 266a stores, for example, a PMID which is a literature ID in PubMed. Here, the PMID is an ID number assigned to each literature by PubMed. The item of title 266b stores, a title of the literature. The item of abstract 266c stores, an abstract of the literature.

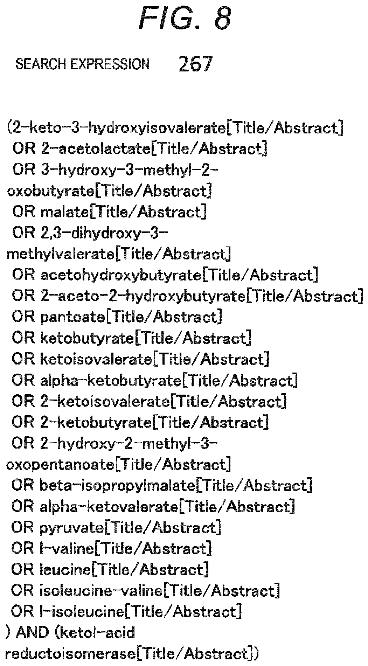

The search expression 267 is, as shown in FIG. 8, a formula representing search information transmitted to the literature search server 300 for the literature search and generated by inputting an input metabolic pathway, and includes information on a substrate of the input metabolic pathway and peripheral compounds including synonymous expressions of the substrate. In this example, grammar is used to search based on the title and abstract of the literature, but a full text search or a search expression for searching for a special item may be used.

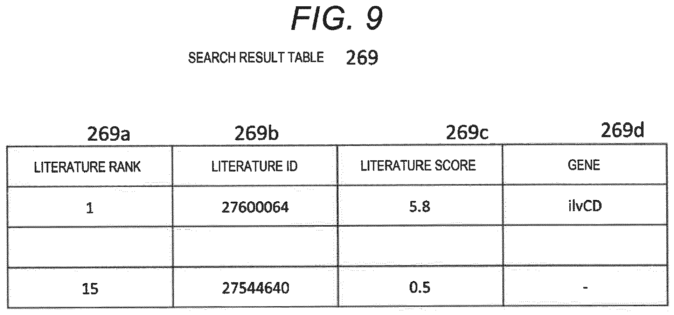

The search result table 269 is a table showing literature information as a search result in a descending order of ranks, and as shown in FIG. 9, includes items of a literature rank 269a, a literature ID 269b, a literature score 269c, and a gene 269d. The item of literature rank 269a stores a rank of the literature according to a literature score. The item of literature ID 269b stores the PMID that is a literature ID corresponding to the literature. The item of literature score 269c stores a literature score (details will be described later) of the literature. The item of gene 269d stores a symbol representing a gene related to an enzyme included in the input metabolic pathway presented in the literature. The example of FIG. 9 shows that a PMID of a literature whose rank is first is 27600064, a literature score is 5.8, and a gene referred to as ilvCD appears in the literature.

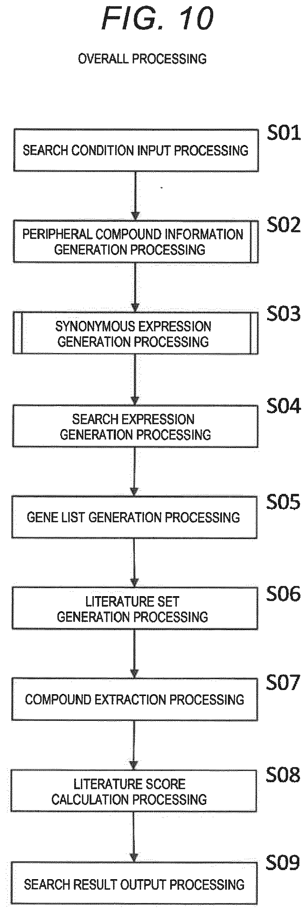

Next, processing of the literature search device will be described with reference to FIG. 10 to FIG. 22.

FIG. 10 is a flowchart showing overall processing of the literature search device from inputting an input metabolic pathway to outputting a literature search result.

Firstly, the overall processing of the literature search device from inputting the input metabolic pathway to outputting the literature search result will be described with reference to FIG. 10.

First, the input metabolic pathway shown in FIG. 4 is input (S01).

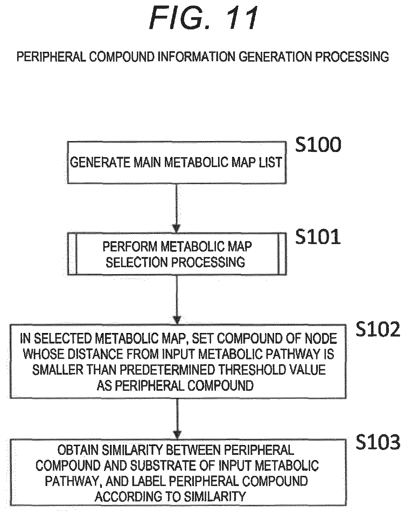

Next, the peripheral compound information generation processing is performed (S02). The processing is processing of generating information on a peripheral compound of the input metabolic pathway. The peripheral compound information generation processing will be described later in detail with reference to FIG. 11.

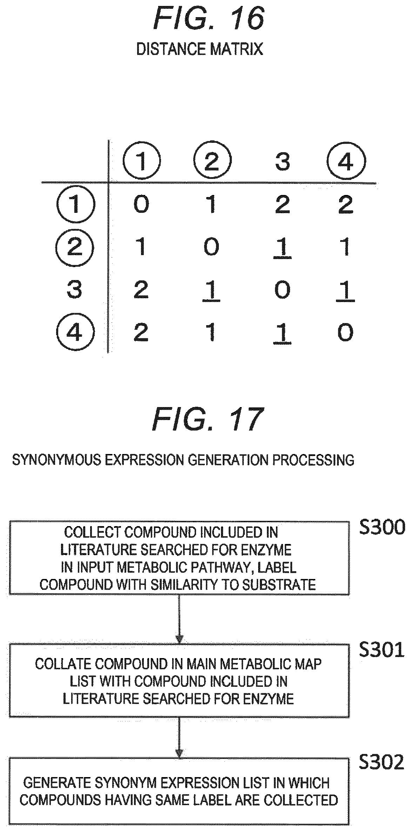

Next, the synonymous expression generation processing is performed (S03). The processing is processing of generating information on a compound that has synonymous expressions of the substrate and the peripheral compound. The synonymous expression generation processing will be described later in detail with reference to FIG. 17.

Next, search expression generation processing is performed (S04). The processing is processing of generating the search expression 267 as shown in FIG. 8 which is input to the search engine 310 in order to search for a literature.

Next, gene list generation processing is performed (S05) . The processing is processing of generating the gene list 265 shown in FIG. 6.

Next, literature set generation processing is performed (S06). The processing is processing of inputting the search expression 267 into the search engine 310, performing a literature search, and generating the literature set list 266 shown in FIG. 7.

Next, compound extraction processing is performed (S07) . The processing is processing of extracting a compound from the titles and abstracts of the literature set list 266.

Next, literature score calculation processing is performed (S08) . The processing is processing of calculating, for each literature, a literature score by taking a sum of the similarity between the compound extracted in the compound extraction processing and the substrate. The literature score calculation processing will be described later in detail.

At last, search result output processing is performed (S09) . The processing is processing of generating the search result table 269 shown in FIG. 9 based on the results obtained so far.

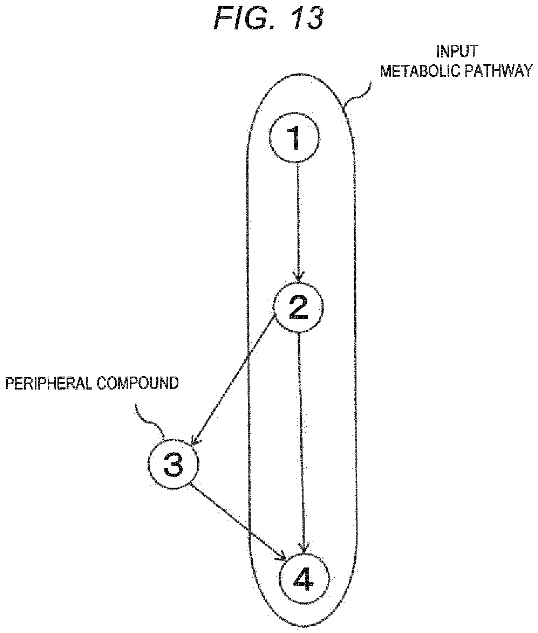

Next, an adjacency matrix and a distance matrix will be described with reference to FIGS. 13 to 16 in order to facilitate understanding of the peripheral compound information generation processing and metabolic map selection processing which is a subroutine of the peripheral compound information generation processing.

Here, an example in which the input metabolic pathway is represented by a part of the metabolic map shown in FIG. 13 will be described. The metabolic map shown in FIG. 13 is node (1) → node (2) → node (4), and further has a graph structure having branches node (2) → node (3), node (3) → node (4). Here, it is assumed that the node (1) → node (2) → node (4) is the input metabolic pathway. A compound corresponding to the node (3) is recognized as a peripheral compound of this input metabolic pathway.

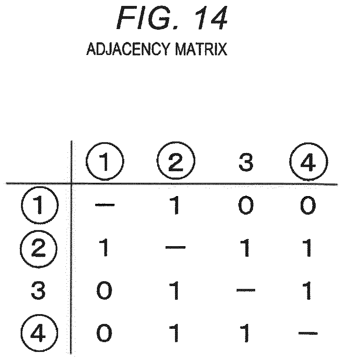

When being represented by an adjacency matrix, the metabolic map of FIG. 13 is as shown in FIG. 14. The adjacency matrix is a matrix showing a connection relationship of respective nodes, in which the nodes of the metabolic map are arranged in a row and a column, when the nodes of the row and the column are neighboring nodes, "1" is set at an intersection, and when the nodes of the row and the column are not neighboring nodes, "0" is set at an intersection. When the nodes of the row and the column are the same, "-" is set at an intersection. In FIG. 14, a node circled by O indicates that the node is a node in the input metabolic pathway.

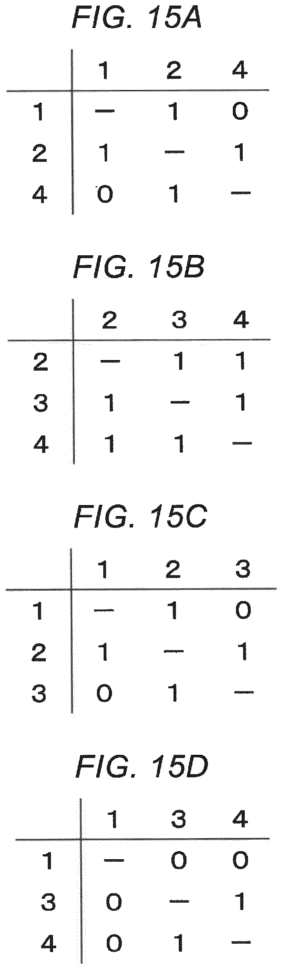

When partial adjacency matrices each having 3 rows and 3 columns are extracted from the adjacency matrix shown in FIG. 14, the partial adjacency matrices are as shown in FIGS. 15A to 15D. The partial adjacency matrices shown in FIGS. 15A to 15D are to be used later in the metabolic map selection processing.

When being represented by a distance matrix, the metabolic map of FIG. 13 is as shown in FIG. 16. The distance matrix is a matrix showing distances between respective nodes, in the matrix, the nodes of the metabolic map are arranged in a row and a column, a distance between the node shown in the row (column) and the node shown in the column (row) is set to "0" when the node shown in the row and the node shown in the column are the same, when the nodes of the row and the column are neighboring nodes, the distance is set to "1", when the nodes of the row and the column sandwich one node, the distance is set to "2", and when the nodes of the row and the column sandwich i nodes (I is an integer equal to or larger than 0), the distance is set to "i+1". Here, the distance between the nodes takes a minimum number of nodes sandwiched therebetween.

Further, the distance between the input metabolic pathway and the node is defined as a minimum value of a distance from the node to the node of the input metabolic pathway. For example, a distance between node (3) and node (1) is "2", and a distance between node (3) and node (2) and a distance between node (3) and node (4) are "1", so that a distance between the node (3) and the input metabolic pathway is "1", which is the minimum value (underlined portion in FIG. 16).

Next, details of the peripheral compound information generation processing will be described with reference to FIGS. 11 to 19.

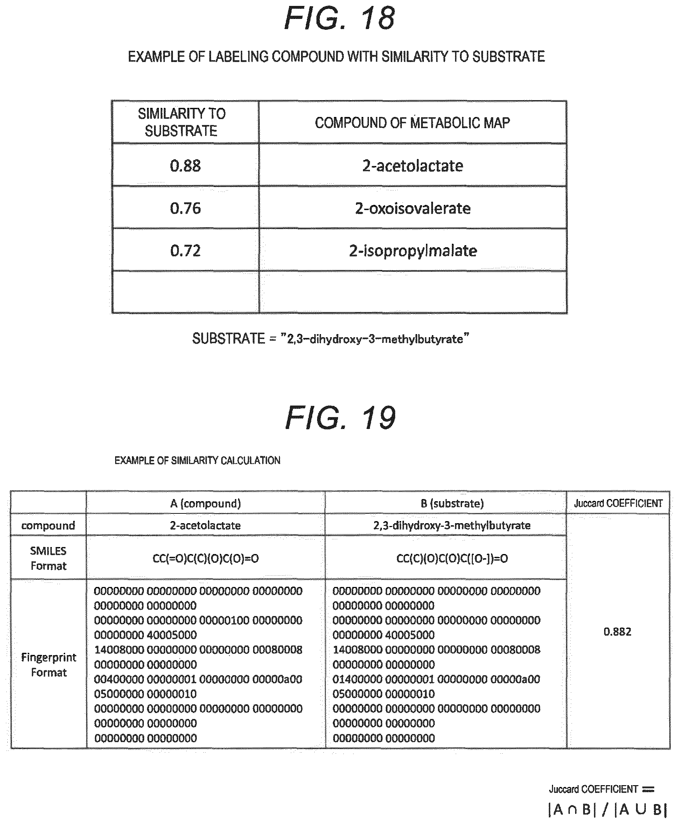

FIG. 18 is a diagram showing an example of labeling the compound with a similarity to the substrate.

FIG. 19 is a diagram showing similarity calculation.

The processing is the processing shown in S02 of FIG. 10.

The peripheral compound information generation processing is processing of setting, as a peripheral compound, a compound on a metabolic pathway in a periphery of a metabolic map of an input metabolic pathway.

Firstly, a main metabolic map list is generated (S100) . In the case of KEGG, some metabolic maps are linked to the item referred to as Pathway on the page searched by the EC number on the publicly available Web page. For example, when the KEGG is searched for the enzyme "EC 4.2.1.9" in the third step shown in FIG. 4, five types of metabolic maps referred to as "EC00290", "EC00770", "EC01100", "EC01110", and "EC01130" can be found. The main metabolic map list is a list of metabolic maps collected by searching for all the enzymes corresponding to the edges of the input metabolic pathway in this way. The main metabolic map list may be manually generated and then may be read into a program, for example, a program that is referred to as a dedicated application interface (API) may be installed in the literature search system, and the main metabolic map may be generated by the dedicated API.

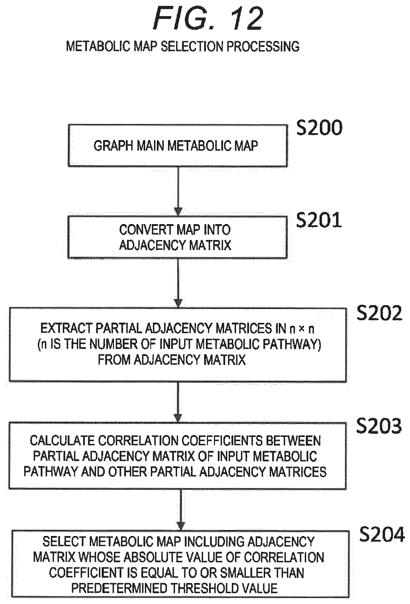

Next, the metabolic map selection processing is performed (S101). The processing is processing of selecting a metabolic map that matches the input metabolic pathway from the main metabolic maps generated in S101. Details of the metabolic map selection processing will be described later with reference to FIG. 12.

Next, in the selected metabolic map, a compound of a node whose distance from the input metabolic pathway is smaller than a predetermined threshold value is set as the peripheral compound (S102).

In the example of FIGS. 13 and 16, the distance of the node (3) from node (1) → node (2) → node (4) in the input metabolic pathway is "1". For example, when the threshold value of the predetermined distance is 2, the node (3) is determined to be the node representing the peripheral compound.

Next, a similarity between the peripheral compound and the substrate of the input metabolic pathway is obtained, and labeling is performed according to the similarity (S103).

Calculation of the similarity will be described with reference to FIG. 19. The similarity is an index showing how the substrate and the compound are structurally similar. In the present embodiment, as an example of the calculation of the similarity, a method of calculating a Juccard coefficient based on a Fingerprint notation will be described.

Firstly, a simplified molecular input line entry system (SMILES) notation of substrates and compounds is determined. The SMILES notation is an unambiguous notation of a structure in which a chemical structure of a molecule is converted into a string of ASCII alphanumeric characters. Then, by the expressed SMILES notation, the Fingerprint notation in each binary expression that is uniquely obtained is obtained. Then, the Juccard coefficient is obtained based on the Fingerprint notation in the binary expression, and is set as the similarity between the substrate and the compound.

The Juccard coefficient is a numerical value obtained by the following Equation 1.

$$ J(A, B) = (|A \cap B| / |A \cup B|) \dots \text{(Equation 1)} $$

Here, $|A \cap B|$ is the number of bits that are the same when compared for each bit, and $|A \cup B|$ is a sum of the number of A bit strings and the number of B bit strings multiplied by 1/2. However, when bit strings of a Fingerprint notation indicating a compound A and a Fingerprint notation indicating a compound B are different, remaining bit strings are different.

In the example shown in FIG. 19, a similarity between the compound A that is "2-acetolactate" and the compound B that is "2,3-dihydroxy-3-methylbutyrate" as a substrate is calculated. For the compound A and the compound B, a first row of the table respectively shows the compounds, a second row respectively shows the SMILES notations of the compounds, and a third row respectively shows the Fingerprint notations of the compounds. The Juccard coefficient is 0.882, which is the similarity between the compound A and the compound B. The Fingerprint notation is a one-dimensional structure descriptor, but the similarity may be calculated by using a high-dimensional structure descriptor. Since as dimension increases, the chemical structure can be described more accurately, it is expected that more accurate similarity can be obtained by calculating the similarity with a high-dimensional structure descriptor.

Then, each compound is labeled by the Juccard coefficient. The labeling means associating a compound with a similarity to a specific substrate. For example, the example in FIG. 18 shows that as an example of the compound, three compounds of "2-acetolactate", "2-oxoisovalerate", and "2-isopropylmalate" are selected, and the three compounds are respectively labeled with similarities between "2,3-dihydroxy-3-methylbutyrate", which is the substrate of the third step shown in FIG. 4, and the three compounds.

Next, the metabolic map selection processing will be described with reference to FIG. 12.

The processing corresponds to the processing shown in S101 of FIG. 11.

Firstly, the main metabolic map is graphed (S200) to form a graph structure as shown in FIG. 13.

Then, the adjacency matrix as shown in FIG. 14 is obtained based on the graph formed in S200 (S201).

Next, n is the number of the input metabolic pathways (in the example shown in FIG. 13 is 3), and from the adjacency matrix represented by the main metabolic map, a partial adjacency matrix in a size of n × n is extracted (S202).

Here, when a metabolic map including the input metabolic pathways is represented by the graph shown in FIG. 13, an adjacency matrix is the adjacency matrix shown in FIG. 14, and when the number n of input metabolic pathways is 3, as shown in FIG. 15, $_4C_3$ = 4 partial adjacency matrices are extracted.

Next, correlation coefficients between the partial adjacency matrix of the input metabolic pathway and other extracted partial adjacency matrices are calculated (S203).

Calculation of the correlation coefficient is performed by the following steps using the partial adjacency matrices shown in FIGS. 15A to 15D. Here, since the input metabolic pathway is node (1) → node (2) → node (4) as shown in FIG. 13, and the partial adjacency matrix representing the input metabolic pathway is as shown in FIG. 15A, correlation coefficients of FIG. 15A and other FIGS. 15B to 15D are obtained.

(Step 1) Convert the partial adjacency matrix into a vector.

For example, a vector of the adjacency matrix in FIG. 15A is as follows.

(-, 1, 0, 1,-, 1, 0, 1,-)

(Step 2) Remove a diagonal component from the transformed vector.

For example, a vector of the adjacency matrix in FIG. 15A is as follows.

(1, 0, 1, 1, 0, 1)

(Step 3) Couple a vector including node values corresponding to columns to the converted vector.

For example, a vector of the adjacency matrix in FIG. 15A is as follows.

(1, 0, 1, 1, 0, 1, 1, 2, 4)

In the description of the present embodiment, an example in which the node value is calculated as a simple numerical value has been described, but when it is assumed in an actual metabolic map, the node value may be a hash value obtained based on a compound name, compound ID or chemical structure corresponding to the node.

Similarly, when (Step 1) to (Step 3) are executed, the vectors shown in FIGS. 15B to 15D are as follows.

(1, 1, 1, 1, 1, 1, 2, 3, 4)
(1, 0, 1, 1, 0, 1, 1, 2, 3)
(0, 0, 0, 1, 0, 1, 1, 3, 4)

(Step 4) Calculate a correlation coefficient (Pearson product moment correlation coefficient) between the vectors.

The correlation coefficient is an index representing the correlation between two types of data variables, and the closer the correlation coefficient is to 1, the more correlative the data to be compared is.

In this example, the respective correlation coefficients are as follows.

A correlation coefficient of the adjacency matrix in FIG. 15A and the adjacency matrix in FIG. 15A is 1.

A correlation coefficient of the adjacency matrix in FIG. 15A and the adjacency matrix in FIG. 15B is 0.8992518.

A correlation coefficient of the adjacency matrix in FIG. 15A and the adjacency matrix in FIG. 15C is 0.9838176.

A correlation coefficient of the adjacency matrix in FIG. 15A and the adjacency matrix in FIG. 15D is 0.9146593.

Next, as a result of the matching, a metabolic map including an adjacency matrix whose absolute value of the correlation coefficient is equal to or smaller than a predetermined threshold value is selected (S204).

For example, when the predetermined threshold value is 0.9, only the metabolic map including the partial adjacency matrix of FIG. 15A is selected.

Next, the synonymous expression generation processing will be described in detail with reference to FIG. 17.

The processing corresponds to the processing shown in S03 of FIG. 10.

The synonymous expression here is a synonymous expression of a compound included in the metabolic map as shown in FIG. 5. For example, a synonymous expression of a compound can be searched by using the dedicated API such as the KEGG. However, here, a method will be described, in which a literature group is determined in advance, and synonymous expressions are selected from compounds that appear in the literature group based on the label of the similarity. Hereinafter, this method will be referred to as a "literature group limitation method". There is no particular restriction on the literature group, and for example, all literatures listed in PubMed may be the literature group described here. In the present embodiment, the literature group is literatures searched for enzymes in the input metabolic pathway shown in FIG. 4. Further, as a method of extracting a compound appearing in a literature, a known method such as a method of collating with a compound dictionary may be applied.

Firstly, compounds included in the literatures searched for the enzymes in the input metabolic pathway are collected based on the literature group limitation method described above, and are labeled with the similarity to the substrate (S300).

Next, the compounds in the main metabolic map list are collated with the compounds included in the literatures searched for the enzymes depending on the label of the similarity (S301).

An example of a collating result is shown in FIG. 18. A first column of the table shows the similarity to the substrate (label of the compound), a second column shows the compound of the metabolic map, and a third column shows the compounds included in the literatures searched by the enzymes alone. In the example shown in FIG. 18, for example, in a first row, a similarity between the compound "2, 3-dihydroxy-3-methylbutyrate" that is the substrate and the compound "2-acetolactate" is 0.88, and thus the labeling is performed.

Next, the synonym expression list 264 in which the compounds having the same label are set as synonymous expressions is generated (S302).

As described above, in the present embodiment, since not only peripheral compounds but also compounds including synonymous expressions that are structurally similar are targeted, more literatures relevant to the input metabolic pathway can be found during searching.

Next, the gene list generation processing will be described in detail.

The processing corresponds to the processing shown in S05 of FIG. 10.

In the gene list generation processing S05, genes corresponding to the enzymes of the input metabolic pathway are collected from a public database and created. Specifically, first, a public database is searched to obtain the EC number of the enzyme. Next, the gene corresponding to the EC number is obtained and a gene list is generated. Here, as public databases, BiGG, Universal Protein Resource (UniProt), and the like are known. For example, when the UniProt is searched for a corresponding gene from an EC number, even a name of an organism that is a host of the gene can be searched for. Similarly to S100, the search processing can be installed in the literature search system by a method of invoking a dedicated API provided by a public database from a program.

Next, the literature set generation processing will be described in detail.

The processing corresponds to the processing shown in S06 of FIG. 10.

In the literature set generation process S06, a literature set is generated by the search expression 267 generated by the search expression generation processing S04. Then, only the literatures in which the genes in the gene list appear are extracted from this literature set, and stored in the literature set list 266 shown in FIG. 7.

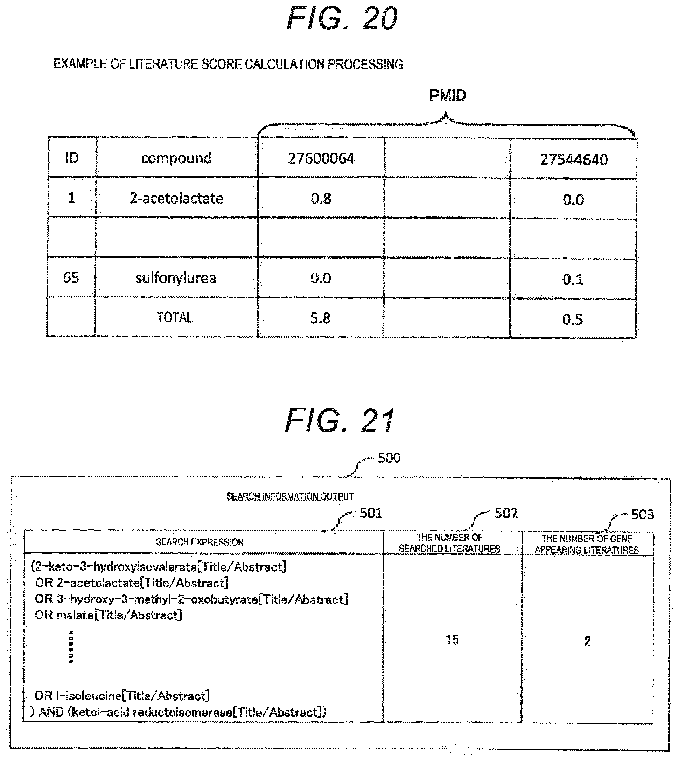

Next, the literature score calculation processing will be described in detail with reference to FIG. 20.

FIG. 20 is a diagram showing the literature score calculation processing.

The processing corresponds to the processing shown in S08 of FIG. 10.

In the literature score calculation processing S08, a similarity (label value) between the compound extracted from the literature set of the literature set list 266 and the substrate is calculated. Here, the substrate is a substrate of the input metabolic pathway shown in FIG. 4. Since a purpose is to label the compounds extracted from the literature set of the literature set list 266, when there are a plurality of substrates as in the example of FIG. 4, one of the substrates may be selected. Then, as shown in FIG. 20, the label values of the compounds appearing in the literature are individually summed in unit of literature to obtain a literature score.

In this way, a high literature score is attached to a literature in which many compounds with a high structural similarity to the substrate of the input metabolic pathway appear, and by ranking the literatures accordingly, the user can easily refer to literatures with higher importance.

In the example shown in FIG. 20, a first column of the table is a compound ID, and in this example, compound IDs of 1 to 65 are entered. Then, a second column of the table is a compound, a third column onward are PMID columns of the literature set of the literature set list 266, and in each column, the similarity (label value) between the compound extracted from the literature set and the substrate is entered. For example, in a first row, "1" is entered as the compound ID, the compound is "2-acetolactate", a label value of a literature whose PMID is 27600064 is 0.8, and a label value of a literature whose PMID is 27544640 is 0.0. In the literature with a PMID of 27544640, the label value is 0.0 because the compound 2-acetolactate does not appear. Then, in a total column at a bottom of the table, a value obtained by summing, for each literature, the label values of the compounds that appear is entered, and this value is set as the literature score. In this example, a literature score of the literature with a PMID of 27600064 is 5.8, and a literature score of the literature with a PMID of 27544640 is 0.5.

The literature score calculation method is not limited to the method described here, and for example, the technique described in JP-A-2002-215672 described above is known. In the case of JP-A-2002-215672, a similarity or a correlation coefficient with all search target literatures of a search target literature group is calculated, and a literature group with a high calculated similarity or a high correlation coefficient is extracted from the search target literature group as a matching literature. That is, it is described that ranking is performed depending on the similarity or the correlation coefficient between literatures. The literature score calculation processing of the present embodiment may adopt such a literature score calculation method as long as reference information suitable for a searcher to determine a priority of a literature reference can be presented. Here, the reference information suitable for the searcher to determine the priority of the literature reference is, for example, the literature rank, the literature score, the gene that appeared in the literature, the organism name that is the host of the gene, the PMID of the literature, and the like.

Next, the search result output processing will be described in detail.

The processing corresponds to the processing shown in S09 of FIG. 10.

In the search result output processing, the literature scores are sorted in a ranking order of the literature score calculation processing S08, and as shown in FIG. 9, the literature information is displayed in the search result table 269 in a descending order of the literature score.

The example of the search result table 269 shown in FIG. 9 shows in the first row that a PMID of the literature ranked first is 27600064, the literature score is 5.8, and the gene referred to as the ilvCD appears.

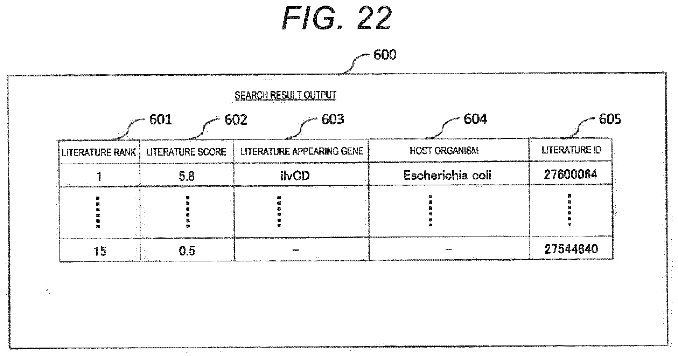

Next, a user interface of the literature search system will be described with reference to FIG. 21 and FIG. 22.

FIG. 21 is a diagram showing an example of a search information output screen.

FIG. 22 is a diagram showing an example of a search result output screen.

A search information output screen 500 is a screen showing search information, and is displayed on the display device 208 of the literature search device 100.

The search information output screen 500 includes, as shown in FIG. 21, a search expression column 501, a literature number column 502, and a gene appearing literature number column 503. The search expression 267 shown in FIG. 8 is displayed in the search expression column 501, the number of literatures in literature sets found by the search expression in the search expression 501 is displayed in the literature number column 502, and the number of literatures in a new literature set created by extracting only the literatures in which genes in the gene list appear from the literature set is displayed in the gene appearing literature number column 503.

A search result output screen 600 is a screen showing search result information of the search result table shown in FIG. 9, and is displayed on the display device 208 of the literature search device 100.

The search result output screen 600 includes, as shown in FIG. 22, a literature rank 601, a literature score 602, a literature appearing gene 603, a host organism 604, and a literature ID 605. Values of the literature rank 269a, the literature score 269c, the gene 269d, and the literature ID 269b in the search result table 269 of FIG. 9 are respectively displayed in the literature rank 601, the literature score 602, the literature appearing gene 603, and the literature ID 605, and a value of the microorganism 265c of the corresponding gene in the gene list of FIG. 6 is displayed in the host organism 604.

As a result, the user can understand the search results of literatures that are ranked and have high importance in association with genes and host organisms.

According to the literature search system of the present embodiment, a literature database is not searched simply based on information of an input metabolic pathway, information on peripheral compounds in a vicinity of a graph of the input metabolic pathway obtained based on an input metabolic map is generated, and literature search is performed based on the search expression including a synonymous expression of the peripheral compound, so that more literature information can be presented to the user as the search result of the literature search. In addition, by searching the related database, information of the gene corresponding to the enzyme of the input metabolic pathway and the microorganism that is the host of the gene can be presented to the user.

**Claims**

1.  An information processing system (100) searching a literature database for a literature, comprising:

    a database (400) including pathway information related to a metabolic pathway that at least includes information on, for each of a plurality of metabolic reactions, a compound produced by reaction, a compound used in reaction, and enzymes that act in the metabolic reaction;
    an input unit (101, 102) configured to receive an input of information on a first reaction compound which was produced by reaction, a first reaction compound which was used in reaction, and a first enzyme that acts in the metabolic reaction for literature search, as pathway information of metabolic reaction for literature search;
    an extraction unit (107) configured to extract, based on the information on the first reaction compound which was produced by reaction, the first reaction compound which was used in reaction, and the first enzyme, pathway information that has a predetermined relationship with the pathway information of metabolic reaction for literature search from the database, and extract, from the extracted pathway information, information on a peripheral compound that is different from the first reaction compound which was produced by reaction and the first reaction compound which was used in reaction; and

a search unit (300) configured to search the literature database (400) for a literature based on the pathway information of metabolic reaction for literature search and the information on the peripheral compound wherein

information related to an input metabolic pathway represented by a directed graph in which a compound that is a reaction compound in a metabolic reaction is set as a node, and an enzyme that acts when a node used in reaction is moved to a node produced by reaction is set as an edge is input, and

a search expression is generated only based on information on the compound and enzyme related to the input metabolic pathway so as to search the literature database (400) for a literature,

**characterized in that**

the peripheral compound is extracted as a compound of a node whose distance on a graph represented by the extracted pathway information with a node of the input metabolic pathway on the graph is smaller than a predetermined threshold value.

2. The information processing system (100) according to the claim 1, wherein
   the pathway information that has a predetermined relationship with the pathway information of metabolic reaction for literature search is pathway information including the first enzyme.

3. The information processing system (100) according to the claim 1, wherein

   a graph represented by the extracted pathway information and the input metabolic pathway are converted into an adjacency matrix representing a connection relationship between nodes of the graph,
   the number of nodes in the input metabolic pathway is set to n, and only a partial adjacency matrix in a size of n × n is extracted from the adjacency matrix representing the graph represented by the extracted pathway information,
   based on the extracted partial adjacency matrix, a correlation coefficient between the partial adjacency matrix of the input metabolic pathway and the other extracted partial adjacency matrix is calculated, a graph including an adjacency matrix whose absolute value of the correlation coefficient is equal to or smaller than a predetermined threshold value is selected, and
   a distance on the graph with the node of the input metabolic pathway is obtained based on a distance matrix of the selected graph, and a compound of a node having a distance smaller than a predetermined threshold is selected as the peripheral compound.

4. The information processing system (100) according to the claim 1, wherein
   a similarity representing a similar degree in chemical structural formula between the compound represented by the node of the input metabolic pathway and the peripheral compound is obtained, a compound having the same similarity as the peripheral compound is set as a compound having a synonymous expression of the peripheral compound, and a search expression is generated based on the compound having the synonymous expression of the peripheral compound.

5. The information processing system (100) according to the claim 1, wherein

   a gene list of genes related to the enzyme of the input metabolic pathway is generated, and
   a literature in which a gene in the gene list appears is selected from the found literature.

6. The information processing system (100) according to the claim 1, wherein

   for each literature, similarities representing similar degrees in chemical structural formula between the compound represented by the node of the input metabolic pathway and the compound appearing in the literature are added when the compound appears in the literature, a sum of the similarities of all compounds is set as a literature score of the literature, and
   the found literature is ranked according to the literature score.

7. The information processing system (100) according to the claim 1, wherein

   a gene related to the enzyme of the input metabolic pathway is obtained, a literature in which the gene appears is selected,
   for each literature, similarities representing similar degrees in chemical structural formula between the compound represented by the node of the input metabolic pathway and the compound appearing in the literature are added when the compound appears in the literature, a sum of the similarities of all compounds is set as a literature score of the literature,

the found literature is ranked according to the literature score, and

a literature ID of the literature, the literature score, and the gene appearing in the literature are displayed according to the literature rank of the literature.

8. The information processing system (100) according to claim 7, further displaying:
a microorganism that is a host of the gene.

9. A search method of searching a literature database (400) of biochemistry for a literature related to input information, the search method comprising:

a step of inputting information (S01) related to an input metabolic pathway represented by a directed graph in which a compound that is a reaction compound in a metabolic reaction is set as a node, and an enzyme that acts when a node used in reaction is moved to a node produced by reaction is set as an edge;

a step of selecting (S101) a main metabolic map including a pathway most similar to or the same as the input metabolic pathway from a database of a metabolic map represented by a directed graph in which a reaction compound in a metabolic reaction is set as a node, and an enzyme that acts when a node used in reaction is moved to a node produced by reaction is set as an edge, and selecting, from the main metabolic map, a compound in a vicinity of the input metabolic pathway as a peripheral compound;

a step of generating (S102) a search expression based on information on the selected peripheral compound and information on the compound and enzyme related to the input metabolic pathway and searching the literature database for a literature,

a step of converting the main metabolic map and the input metabolic pathway into an adjacency matrix representing a connection relationship between nodes of the graph;

a step of setting the number of nodes in the input metabolic pathway to n, and extracting a partial adjacency matrix in a size of $n \times n$ from the adjacency matrix representing the graph represented by the extracted pathway information;

a step of calculating, based on the extracted partial adjacency matrix, a correlation coefficient between the partial adjacency matrix of the input metabolic pathway and the other extracted partial adjacency matrix, and selecting a metabolic map including an adjacency matrix whose absolute value of the correlation coefficient is equal to or smaller than a predetermined threshold value; and

a step of obtaining (S07) a distance on the graph with the node of the input metabolic pathway based on a distance matrix of the selected metabolic map, and selecting a compound of a node having a distance smaller than a predetermined threshold as the peripheral compound.

10. The search method according to claim 9, further comprising:
a step of obtaining (S103) a similarity representing a similar degree in chemical structural formula between the compound represented by the node of the input metabolic pathway and the peripheral compound, setting a compound having the same similarity as the peripheral compound as a compound having a synonymous expression of the peripheral compound, and then generating a search expression based on the compound having the synonymous expression of the peripheral compound.

11. The search method according to claim 9, further comprising:

a step of generating a gene list (S05) of genes related to the enzyme of the input metabolic pathway; and
a step of selecting a literature (S06) in which a gene in the gene list appears from the found literature.

12. The search method according to claim 9, further comprising:

a step of obtaining a gene related to the enzyme of the input metabolic pathway, and selecting a literature in which the gene appears;

a step of adding, for each literature, similarities representing similar degrees in chemical structural formula between the compound represented by the node of the input metabolic pathway and the compound appearing in the literature when the compound appears in the literature, setting a sum of the similarities of all compounds as a literature score of the literature; and

a step of ranking the found literature according to the literature score.

**Patentansprüche**

1. Datenverarbeitungssystem (100), das eine Literaturdatenbank nach einer Literatur durchsucht und das Folgendes umfasst:

   eine Datenbank (400), die Weginformationen enthält, die mit einem Stoffwechselweg in Beziehung stehen, der mindestens für jede von mehreren Stoffwechselreaktionen Informationen über eine Verbindung, die durch eine Reaktion erzeugt wird, eine Verbindung, die in einer Reaktion verwendet wird, und Enzyme, die in der Stoffwechselreaktion auftreten, enthält;

   eine Eingabeeinheit (101, 102), die konfiguriert ist, eine Eingabe von Informationen über eine erste Reaktionsverbindung, die durch eine Reaktion erzeugt wurde, eine erste Reaktionsverbindung, die in einer Reaktion verwendet wurde, und ein erstes Enzym, das in der Stoffwechselreaktion zur Literatursuche wirkt, als Weginformationen einer Stoffwechselreaktion zur Literatursuche aufzunehmen;

   eine Extraktionseinheit (107), die konfiguriert ist, Weginformationen, die eine vorgegebene Beziehung zu den Weginformationen einer Stoffwechselreaktion zur Literatursuche besitzen, auf der Grundlage der Informationen über die erste Reaktionsverbindung, die durch eine Reaktion erzeugt wurde, die erste Reaktionsverbindung, die in einer Reaktion verwendet wurde, und das erste Enzym aus der Datenbank zu extrahieren und aus den extrahierten Weginformationen Informationen über eine Peripherieverbindung, die von der ersten Reaktionsverbindung, die durch eine Reaktion erzeugt wurde, und der ersten Reaktionsverbindung, die in einer Reaktion verwendet wurde, verschieden sind, zu extrahieren; und

   eine Sucheinheit (300), die konfiguriert ist, auf der Grundlage der Weginformationen einer Stoffwechselreaktion zur Literatursuche und der Informationen über die Peripherieverbindung die Literaturdatenbank (400) nach einer Literatur zu durchsuchen, wobei

   Informationen, die mit einem eingegebenen Stoffwechselweg, der durch einen gerichteten Graphen repräsentiert ist, in dem eine Verbindung, die eine Reaktionsverbindung in einer Stoffwechselreaktion ist, als ein Knoten gesetzt ist, und ein Enzym, das wirkt, wenn ein Knoten, der in einer Reaktion verwendet wird, zu einem Knoten, der durch eine Reaktion erzeugt wird, versetzt wird, als eine Kante gesetzt ist, in Beziehung stehen, eingegeben werden und

   ein Suchausdruck lediglich auf der Grundlage von Informationen über die Verbindung und das Enzym, die mit dem eingegebenen Stoffwechselweg in Beziehung stehen, erzeugt wird, um die Literaturdatenbank (400) nach einer Literatur zu durchsuchen,

   **dadurch gekennzeichnet, dass**

   die Peripherieverbindung als eine Verbindung eines Knotens, dessen Entfernung in einem Graphen, der durch die extrahierten Weginformationen repräsentiert ist, zu einem Knoten des eingegebenen Stoffwechselwegs im Graphen kleiner als ein vorgegebener Schwellenwert ist, extrahiert wird.

2. Datenverarbeitungssystem (100) nach Anspruch 1, wobei
   die Weginformationen, die eine vorgegebene Beziehung zu den Weginformationen einer Stoffwechselreaktion zur Literatursuche besitzen, Weginformationen sind, die das erste Enzym enthalten.

3. Datenverarbeitungssystem (100) nach Anspruch 1, wobei

   ein Graph, der durch die extrahierten Weginformationen und den eingegebenen Stoffwechselweg repräsentiert ist, in eine Adjazenzmatrix umgewandelt wird, die eine Verbindungsbeziehung zwischen Knoten des Graphen repräsentiert,

   die Anzahl von Knoten im eingegebenen Stoffwechselweg zu n gesetzt ist und lediglich eine Teiladjazenzmatrix einer Größe $n \times n$ aus der Adjazenzmatrix, die den Graphen, der durch die extrahierten Weginformationen repräsentiert ist, repräsentiert, extrahiert wird,

   ein Korrelationskoeffizient zwischen der Teiladjazenzmatrix des eingegebenen Stoffwechselwegs und der weiteren extrahierten Teiladjazenzmatrix auf der Grundlage der extrahierten Teiladjazenzmatrix berechnet wird und ein Graph, der eine Adjazenzmatrix enthält, deren Betrag des Korrelationskoeffizienten gleich oder kleiner als ein vorgegebener Schwellenwert ist, gewählt wird und

   eine Entfernung im Graphen zum Knoten des eingegebenen Stoffwechselwegs auf der Grundlage einer Entfernungsmatrix des gewählten Graphen erhalten wird und eine Verbindung eines Knotens, der eine Entfernung aufweist, die kleiner als ein vorgegebener Schwellenwert ist, als die Peripherieverbindung gewählt wird.

4. Datenverarbeitungssystem (100) nach Anspruch 1, wobei
   eine Ähnlichkeit, die einen ähnlichen Grad einer chemischen Strukturformel zwischen der Verbindung, die durch den Knoten des eingegebenen Stoffwechselwegs repräsentiert ist, und der Peripherieverbindung repräsentiert, erhalten wird, eine Verbindung, die dieselbe Ähnlichkeit wie die Peripherieverbindung aufweist, als eine Verbindung, die einen synonymen Ausdruck der Peripherieverbindung aufweist, gesetzt wird und ein Suchausdruck auf der Grundlage der Verbindung, die den synonymen Ausdruck der Peripherieverbindung aufweist, erzeugt wird.

5. Datenverarbeitungssystem (100) nach Anspruch 1, wobei

eine Genliste von Genen, die mit dem Enzym des eingegebenen Stoffwechselwegs in Beziehung stehen, erzeugt wird und
eine Literatur, in der ein Gen aus der Genliste erscheint, aus der gefundenen Literatur gewählt wird.

6. Datenverarbeitungssystem (100) nach Anspruch 1, wobei

für jede Literatur Ähnlichkeiten, die ähnliche Grade chemischer Strukturformeln zwischen der Verbindung, die durch den Knoten des eingegebenen Stoffwechselwegs repräsentiert ist, und der Verbindung, die in der Literatur erscheint, repräsentieren, addiert werden, wenn die Verbindung in der Literatur erscheint, und eine Summe der Ähnlichkeiten aller Verbindungen als eine Literaturpunktzahl der Literatur gesetzt wird, und
die gefundene Literatur gemäß der Literaturpunktzahl eingestuft wird.

7. Datenverarbeitungssystem (100) nach Anspruch 1, wobei

ein Gen, das mit dem Enzym des eingegebenen Stoffwechselwegs in Beziehung steht, erhalten wird und eine Literatur, in der das Gen erscheint, gewählt wird,
für jede Literatur Ähnlichkeiten, die ähnliche Grade chemischer Strukturformeln zwischen der Verbindung, die durch den Knoten des eingegebenen Stoffwechselwegs repräsentiert ist, und der Verbindung, die in der Literatur erscheint, repräsentieren, addiert werden, wenn die Verbindung in der Literatur erscheint, und eine Summe der Ähnlichkeiten aller Verbindungen als eine Literaturpunktzahl der Literatur gesetzt wird,
die gefundene Literatur gemäß der Literaturpunktzahl eingestuft wird und
eine Literaturkennung der Literatur, die Literaturpunktzahl und das Gen, das in der Literatur erscheint, gemäß der Literatureinstufung der Literatur angezeigt werden.

8. Datenverarbeitungssystem (100) nach Anspruch 7, das ferner Folgendes zeigt:
einen Mikroorganismus, der ein Wirt des Gens ist.

9. Suchverfahren zum Durchsuchen einer Literaturdatenbank (400) der Biochemie nach einer Literatur, die mit eingegebenen Informationen in Beziehung steht, wobei das Suchverfahren Folgendes umfasst:

einen Schritt (S01) des Eingebens von Informationen, die mit einem eingegebenen Stoffwechselweg, der durch einen gerichteten Graphen repräsentiert ist, in dem eine Verbindung, die eine Reaktionsverbindung in einer Stoffwechselreaktion ist, als ein Knoten gesetzt ist, und ein Enzym, das wirkt, wenn ein Knoten, der in einer Reaktion verwendet wird, zu einem Knoten, der durch eine Reaktion erzeugt wird, versetzt wird, als eine Kante gesetzt ist, in Beziehung stehen;
einen Schritt (S101) des Wählens einer Hauptstoffwechselkarte, die einen Pfad enthält, der am ähnlichsten oder gleich dem eingegebenen Stoffwechselweg ist, aus einer Datenbank einer Stoffwechselkarte, die durch einen gerichteten Graphen repräsentiert ist, in dem eine Reaktionsverbindung in einer Stoffwechselreaktion als ein Knoten gesetzt ist, und ein Enzym, das wirkt, wenn ein Knoten, der in einer Reaktion verwendet wird, zu einem Knoten, der durch eine Reaktion erzeugt wird, versetzt wird, als eine Kante gesetzt ist, und des Wählens aus der Hauptstoffwechselkarte einer Verbindung in einer Umgebung des eingegebenen Stoffwechselwegs als eine Peripherieverbindung;
einen Schritt (S102) des Erzeugens eines Suchausdrucks auf der Grundlage von Informationen über die gewählte Peripherieverbindung und Informationen über die Verbindung und das Enzym, die mit dem eingegebenen Stoffwechselweg in Beziehung stehen, und des Durchsuchens der Literaturdatenbank nach einer Literatur,
einen Schritt des Umwandelns der Hauptstoffwechselkarte und des eingegebenen Stoffwechselwegs in eine Adjazenzmatrix, die eine Verbindungsbeziehung zwischen Knoten des Graphen repräsentiert;
einen Schritt des Setzens der Anzahl von Knoten im eingegebenen Stoffwechselweg zu n und des Extrahierens einer Teiladjazenzmatrix einer Größe $n \times n$ aus der Adjazenzmatrix, die den Graphen, der durch die extrahierten Weginformationen repräsentiert ist, repräsentiert;
einen Schritt des Berechnens auf der Grundlage der extrahierten Teiladjazenzmatrix eines Korrelationskoeffizienten zwischen der Teiladjazenzmatrix des eingegebenen Stoffwechselwegs und der weiteren extrahierten Teiladjazenzmatrix und des Wählens einer Stoffwechselkarte, die eine Adjazenzmatrix enthält, deren Betrag des Korrelationskoeffizienten gleich oder kleiner als ein vorgegebener Schwellenwert ist; und einen Schritt (SO7) des Erhaltens einer Entfernung im Graphen zum Knoten des eingegebenen Stoffwechselwegs auf der Grundlage einer Entfernungsmatrix der gewählten Stoffwechselkarte und des Wählens einer Verbindung eines Knotens, der eine Entfernung aufweist, die kleiner als ein vorgegebener Schwellenwert ist, als die Peripherieverbindung.

10. Suchverfahren nach Anspruch 9, das ferner Folgendes umfasst:
einen Schritt (S103) des Erhaltens einer Ähnlichkeit, die einen ähnlichen Grad einer chemischen Strukturformel zwischen der Verbindung, die durch den Knoten des eingegebenen Stoffwechselwegs repräsentiert ist, und der Peripherieverbindung repräsentiert, des Setzens einer Verbindung, die dieselbe Ähnlichkeit wie die Peripherieverbindung aufweist, als eine Verbindung, die einen synonymen Ausdruck der Peripherieverbindung aufweist, und dann des Erzeugens eines Suchausdrucks auf der Grundlage der Verbindung, die den synonymen Ausdruck der Peripherieverbindung aufweist.

11. Suchverfahren nach Anspruch 9, das ferner Folgendes umfasst:

einen Schritt (S05) des Erzeugens einer Genliste von Genen, die mit dem Enzym des eingegebenen Stoffwechselwegs in Beziehung stehen; und
einen Schritt (S06) des Wählens einer Literatur, in der ein Gen aus der Genliste erscheint, aus der gefundenen Literatur.

12. Suchverfahren nach Anspruch 9, das ferner Folgendes umfasst:

einen Schritt des Erhaltens eines Gens, das mit dem Enzym des eingegebenen Stoffwechselwegs in Beziehung steht, und des Wählens einer Literatur, in der das Gen erscheint;
einen Schritt des Addierens für jede Literatur von Ähnlichkeiten, die ähnliche Grade chemischer Strukturformeln zwischen der Verbindung, die durch den Knoten des eingegebenen Stoffwechselwegs repräsentiert ist, und der Verbindung, die in der Literatur erscheint, repräsentieren, wenn die Verbindung in der Literatur erscheint, und des Setzens einer Summe der Ähnlichkeiten aller Verbindungen als eine Literaturpunktzahl der Literatur und einen Schritt des Einstufens der gefundenen Literatur gemäß der Literaturpunktzahl.

## Revendications

1. Système de traitement d'informations (100) recherchant une documentation dans une base de données de documentation, comprenant :

une base de données (400) incluant des informations de voie en lien avec une voie métabolique qui inclut au moins des informations concernant, pour chacune d'une pluralité de réactions métaboliques, un composé produit par réaction, un composé utilisé en réaction, et des enzymes qui agissent dans la réaction métabolique ;
une unité d'entrée (101, 102) configurée pour recevoir une entrée d'informations concernant un premier composé de réaction qui a été produit par réaction, un premier composé de réaction qui a été utilisé en réaction, et un premier enzyme qui agit dans la réaction métabolique pour une recherche de documentation, à titre d'informations de voie de réaction métabolique pour une recherche de documentation ;
une unité d'extraction (107) configurée pour extraire, sur la base des informations concernant le premier composé de réaction qui a été produit par réaction, le premier composé de réaction qui a été utilisé en réaction, et le premier enzyme, des informations de voie qui ont une relation prédéterminée avec les informations de voie de réaction métabolique pour une recherche de documentation à partir de la base de données, et pour extraire, à partir des informations de voie extraites, des informations concernant un composé périphérique qui est différent du premier composé de réaction qui a été produit par réaction et du premier composé de réaction qui a été utilisée en réaction ; et
une unité de recherche (300) configurée pour rechercher une documentation dans la base de données de documentation (400) sur la base des informations de voie de réaction métabolique pour une recherche de documentation et des informations concernant le composé périphérique,
dans lequel
des informations en lien avec une voie métabolique d'entrée représentées par un graphique dirigé dans lequel un composé qui est un composé de réaction dans une réaction métabolique est défini à titre de nœud, et un enzyme qui agit quand un nœud utilisé en réaction est déplacé jusqu'à un nœud produit par réaction est défini à titre de bord, sont entrées, et

une expression de recherche est générée uniquement sur la base d'informations concernant le composé et l'enzyme en lien avec la voie métabolique d'entrée de manière à rechercher une documentation dans la base de données de documentation (400),

**caractérisé en ce que**

le composé périphérique est extrait à titre de composé d'un nœud dont une distance sur un graphique représentée par les informations de voie extraites avec un nœud de la voie métabolique d'entrée sur le graphique est plus petite qu'une valeur seuil prédéterminée.

2. Système de traitement d'informations (100) selon la revendication 1, dans lequel
les informations de voie qui ont une relation prédéterminée avec les informations de voie de réaction métabolique pour une recherche de documentation sont des informations de voie incluant le premier enzyme.

3. Système de traitement d'informations (100) selon la revendication 1, dans lequel

un graphique représenté par les informations de voie extraites et la voie métabolique d'entrée sont convertis en une matrice d'adjacence représentant une relation de connexion entre des nœuds du graphique,
le nombre de nœuds dans la voie métabolique d'entrée est défini à n, et seule une matrice d'adjacence partielle dans une taille de n × n est extraite de la matrice d'adjacence représentant le graphique représenté par les informations de voie extraites,
sur la base de la matrice d'adjacence partielle extraite, un coefficient de corrélation entre la matrice d'adjacence partielle de la voie métabolique d'entrée et l'autre matrice d'adjacence partielle extraite est calculé, un graphique incluant une matrice d'adjacence dont une valeur absolue du coefficient de corrélation est égale ou inférieure à une valeur seuil prédéterminée est sélectionnée, et
une distance sur le graphique avec le nœud de la voie métabolique d'entrée est obtenue sur la base d'une matrice de distance du graphique sélectionné, et un composé d'un nœud ayant une distance inférieure à un seuil prédéterminé est sélectionné à titre de composé périphérique.

4. Système de traitement d'informations (100) selon la revendication 1, dans lequel
une similarité représentant un degré de similarité dans une formule structurelle chimique entre le composé représenté par le nœud de la voie métabolique d'entrée et le composé périphérique est obtenu, un composé ayant la même similarité que le composé périphérique est défini à titre de composé ayant une expression synonyme du composé périphérique, et une expression de recherche générée sur la base du composé ayant l'expression synonyme du composé périphérique.

5. Système de traitement d'informations (100) selon la revendication 1, dans lequel

une liste de gènes de gènes en lien avec l'enzyme de la voie métabolique d'entrée est générée, et
une documentation dans laquelle un gène dans la liste de gènes apparaît est sélectionnée à partir de la documentation trouvée.

6. Système de traitement d'informations (100) selon la revendication 1, dans lequel

pour chaque documentation, des similarités représentant des degrés de similarité dans une formule structurelle chimique entre le composé représenté par le nœud de la voie métabolique d'entrée et le composé apparaissant dans la documentation sont ajoutées quand le composé apparaît dans la documentation, une somme des similarités de tous les composés est définie à titre de résultat de documentation de la documentation, et
la documentation trouvée est classée en accord avec le résultat de documentation.

7. Système de traitement d'informations (100) selon la revendication 1, dans lequel

un gène en lien avec l'enzyme de la voie métabolique d'entrée est obtenu, une documentation dans laquelle le gène apparaît est sélectionnée,
pour chaque documentation, des similarités représentant des degrés de similarité dans une formule structurelle chimique entre le composé représenté par le nœud de la voie métabolique d'entrée et le composé apparaissant dans la documentation sont ajoutées quand le composé apparaît dans la documentation, une somme des similarités de tous les composés est définie à titre de résultat de documentation de la documentation, la documentation trouvée est classée en accord avec le résultat de documentation, et un ID de documentation de la documentation, le résultat de documentation, et le gène apparaissant dans la documentation sont affichés en accord avec le classement de documentation de la documentation.

8. Système de traitement d'informations (100) selon la revendication 7, affichant en outre : micro-organisme qui est un hôte du gène.

9. Procédé de recherche consistant à rechercher une documentation dans une base de données de documentation (400) de biochimie, en lien avec des informations d'entrée, le procédé de recherche comprenant :

une étape consistant à entrer des informations (S01) en lien avec une voie métabolique d'entrée représentée par un graphique dans lequel un composé qui est un composé de réaction dans une réaction métabolique est défini à titre de nœud, et un enzyme qui agit quand un nœud utilisé en réaction est déplacé jusqu'à un nœud produit par réaction est défini à titre de bord ;
une étape consistant à sélectionner (S101) une carte métabolique principale incluant une voie la plus similaire ou identique à la voie métabolique d'entrée à partir d'une base de données d'une carte métabolique représentée par un graphique dirigé dans lequel un composé de réaction dans une réaction métabolique est défini à titre de nœud, et un enzyme qui agit quand un nœud utilisé en réaction est déplacé jusqu'à un nœud produit par réaction est défini à titre de bord, et à sélectionner, à partir de la carte métabolique principale, un composé dans un voisinage de la voie métabolique d'entrée à titre de composé périphérique ;
une étape consistant à générer (S102) une expression de recherche sur la base des informations concernant le composé périphérique sélectionné et des informations concernant le composé et l'enzyme en lien avec la voie métabolique d'entrée, et à rechercher une documentation dans une base de données de documentation,
une l'étape consistant à convertir la carte métabolique principale et la voie métabolique d'entrée en une matrice d'adjacence représentant une relation de connexion entre des nœuds du graphique,
une étape consistant à définir le nombre de nœuds dans la voie métabolique d'entrée à n, et à extraire une matrice d'adjacence partielle dans une taille de $n \times n$ à partir de la matrice d'adjacence représentant le graphique représenté par les informations de voie extraites,
une l'étape consistant à calculer, sur la base de la matrice d'adjacence partielle extraite, un coefficient de corrélation entre la matrice d'adjacence partielle de la voie métabolique d'entrée et l'autre matrice d'adjacence partielle extraite, et à sélectionner une carte métabolique incluant une matrice d'adjacence dont une valeur absolue du coefficient de corrélation est égale ou inférieure à une valeur seuil prédéterminée ; et
une étape consistant à obtenir (S07) une distance sur le graphique avec le nœud de la voie métabolique d'entrée sur la base d'une matrice de distance de la carte métabolique sélectionnée, et à sélectionner un composé d'un nœud ayant une distance inférieure à un seuil prédéterminé à titre de composé périphérique.

10. Procédé de recherche selon la revendication 9, dans lequel une étape consistant à obtenir (S103) une similarité représentant un degré de similarité dans une formule structurelle chimique entre le composé représenté par le nœud de la voie métabolique d'entrée et le composé périphérique, à définir un composé ayant la même similarité que le composé périphérique à titre de composé ayant une expression synonyme du composé périphérique, et puis à générer une expression de recherche sur la base du composé ayant l'expression synonyme du composé périphérique.

11. Procédé de recherche selon la revendication 9, comprenant en outre :

une étape consistant à générer une liste de gènes (S05) de gènes en lien avec l'enzyme de la voie métabolique d'entrée ; et
une étape consistant à sélectionner une documentation (S06) dans laquelle un gène dans la liste de gènes apparaît à partir de la documentation trouvée.

12. Procédé de recherche selon la revendication 9, comprenant en outre :

une étape consistant à obtenir un gène en lien avec l'enzyme de la voie métabolique d'entrée, et à sélectionner une documentation dans laquelle gène apparaît ;
une étape consistant à ajouter, pour chaque documentation, des similarités représentant des degrés de similarité dans une formule structurelle chimique entre le composé représenté par le nœud de la voie métabolique d'entrée et le composé apparaissant dans la documentation quand le composé apparaît dans la documentation, à définir une somme des similarités de tous les composés à titre de résultat de documentation de la documentation ; et
une étape consistant à classer la documentation trouvée en accord avec le résultat de documentation.

# FIG. 1

**300**

LITERATURE
SEARCH SERVER
**310**

SEARCH ENGINE

**270**
LITERATURE DATABASE

**5**

**400**
METABOLIC MAP
DATABASE SERVER

**261**
METABOLIC MAP

**100**
LITERATURE SEARCH DEVICE

METABOLIC PATHWAY
INPUT UNIT **101**

PERIPHERAL COMPOUND
INFORMATION GENERATION UNIT **102**

SYNONYMOUS EXPRESSION
GENERATION UNIT **103**

SEARCH EXPRESSION
GENERATION UNIT **104**

GENE LIST GENERATION UNIT **105**

LITERATURE SET
GENERATION UNIT **106**

COMPOUND EXTRACTION UNIT **107**

LITERATURE SCORE
CALCULATION UNIT **108**

SEARCH RESULT OUTPUT UNIT **109**

STORAGE UNIT **120**

# FIG. 2

LITERATURE SEARCH DEVICE   100

**201** CPU

**202** ROM

**203** RAM

**204** NETWORK I/F

**210**

**205** INPUT/ OUTPUT I/F

**220** AUXILIARY STORAGE I/F

**208** DISPLAY DEVICE

**207** GRAPHIC CONTROLLER

**206** OPERATION DEVICE

**230** HDD

# FIG. 3

**230**

## HDD

| METABOLIC PATHWAY INPUT PROGRAM | 241 |
| PERIPHERAL COMPOUND INFORMATION GENERATION PROGRAM | 242 |
| SYNONYMOUS EXPRESSION GENERATION PROGRAM | 243 |
| SEARCH EXPRESSION GENERATION PROGRAM | 244 |
| GENE LIST GENERATION PROGRAM | 245 |
| LITERATURE SET GENERATION PROGRAM | 246 |
| COMPOUND EXTRACTION PROGRAM | 247 |
| LITERATURE SCORE CALCULATION PROGRAM | 248 |
| SEARCH RESULT OUTPUT PROGRAM | 249 |

**230**

## HDD

| SYNONYMOUS EXPRESSION LIST | 264 |
| GENE LIST | 265 |
| LITERATURE SET LIST | 266 |
| SEARCH EXPRESSION | 267 |
| SEARCH RESULT TABLE | 269 |

# FIG. 4

INPUT METABOLIC PATHWAY  260

METABOLIC PATHWAY

| FIRST STEP | SECOND STEP | THIRD STEP |
|---|---|---|

SUBSTRATE "C00022"    SUBSTRATE "C00900"    SUBSTRATE "C04039"    isobutanol

EC "2. 2. 1. 6"    EC "1. 1. 1. 86"    EC "4. 2. 1. 9"

22

# FIG. 5

METABOLIC MAP (map00290)     261

# FIG. 6

GENE LIST    265

|  | 265a | 265b | 265c |
| --- | --- | --- | --- |
| | EC NUMBER | GENE | MICROORGANISM |
| | 2.2.1.6 | ilvB1 | Mycobacterium tuberculosis |
| | 2.2.1.6 | ilvG | Escherichia coli (strain K12) |
| | 1.1.1.86 | ilvCD | Escherichia coli |
| | | | |

# FIG. 7

LITERATURE SET LIST    266

| 266a | 266b | 266c |
| --- | --- | --- |
| PMID | TITLE | ABSTRACT |
| 27600064 | Biosynthesis of poly(2-hydroxyisovalerate-co-lactate) ... | Polyhydroxyalkanoates (PHAs) containing 2-hydroxyacids such as ... |
| | | |
| 27544640 | A proteomic analysis of salt stress response ... | Salt stress is one of the key abiotic stresses threatening future agricultural ... |

# FIG. 8

SEARCH EXPRESSION    267

(2-keto-3-hydroxyisovalerate[Title/Abstract]
 OR 2-acetolactate[Title/Abstract]
 OR 3-hydroxy-3-methyl-2-
oxobutyrate[Title/Abstract]
 OR malate[Title/Abstract]
 OR 2,3-dihydroxy-3-
methylvalerate[Title/Abstract]
 OR acetohydroxybutyrate[Title/Abstract]
 OR 2-aceto-2-hydroxybutyrate[Title/Abstract]
 OR pantoate[Title/Abstract]
 OR ketobutyrate[Title/Abstract]
 OR ketoisovalerate[Title/Abstract]
 OR alpha-ketobutyrate[Title/Abstract]
 OR 2-ketoisovalerate[Title/Abstract]
 OR 2-ketobutyrate[Title/Abstract]
 OR 2-hydroxy-2-methyl-3-
oxopentanoate[Title/Abstract]
 OR beta-isopropylmalate[Title/Abstract]
 OR alpha-ketovalerate[Title/Abstract]
 OR pyruvate[Title/Abstract]
 OR l-valine[Title/Abstract]
 OR leucine[Title/Abstract]
 OR isoleucine-valine[Title/Abstract]
 OR l-isoleucine[Title/Abstract]
) AND (ketol-acid
reductoisomerase[Title/Abstract])

# FIG. 9

SEARCH RESULT TABLE  269

| 269a | 269b | 269c | 269d |
|---|---|---|---|
| LITERATURE RANK | LITERATURE ID | LITERATURE SCORE | GENE |
| 1 | 27600064 | 5.8 | ilvCD |
|  |  |  |  |
| 15 | 27544640 | 0.5 | - |

# FIG. 10

OVERALL PROCESSING

```
┌─────────────────────────────────────┐  S01
│  SEARCH CONDITION INPUT PROCESSING   │
└─────────────────────────────────────┘
                   │
                   ▼
┌─────────────────────────────────────┐  S02
│  PERIPHERAL COMPOUND INFORMATION     │
│      GENERATION PROCESSING           │
└─────────────────────────────────────┘
                   │
                   ▼
┌─────────────────────────────────────┐  S03
│      SYNONYMOUS EXPRESSION           │
│      GENERATION PROCESSING           │
└─────────────────────────────────────┘
                   │
                   ▼
┌─────────────────────────────────────┐  S04
│       SEARCH EXPRESSION              │
│      GENERATION PROCESSING           │
└─────────────────────────────────────┘
                   │
                   ▼
┌─────────────────────────────────────┐  S05
│   GENE LIST GENERATION PROCESSING    │
└─────────────────────────────────────┘
                   │
                   ▼
┌─────────────────────────────────────┐  S06
│       LITERATURE SET                 │
│      GENERATION PROCESSING           │
└─────────────────────────────────────┘
                   │
                   ▼
┌─────────────────────────────────────┐  S07
│   COMPOUND EXTRACTION PROCESSING     │
└─────────────────────────────────────┘
                   │
                   ▼
┌─────────────────────────────────────┐  S08
│      LITERATURE SCORE                │
│     CALCULATION PROCESSING           │
└─────────────────────────────────────┘
                   │
                   ▼
┌─────────────────────────────────────┐  S09
│   SEARCH RESULT OUTPUT PROCESSING    │
└─────────────────────────────────────┘
```

# FIG. 11

PERIPHERAL COMPOUND INFORMATION GENERATION PROCESSING

GENERATE MAIN METABOLIC MAP LIST — S100

PERFORM METABOLIC MAP
SELECTION PROCESSING — S101

IN SELECTED METABOLIC MAP, SET COMPOUND OF NODE
WHOSE DISTANCE FROM INPUT METABOLIC PATHWAY IS
SMALLER THAN PREDETERMINED THRESHOLD VALUE
AS PERIPHERAL COMPOUND — S102

OBTAIN SIMILARITY BETWEEN PERIPHERAL
COMPOUND AND SUBSTRATE OF INPUT METABOLIC
PATHWAY, AND LABEL PERIPHERAL COMPOUND
ACCORDING TO SIMILARITY — S103

# FIG. 12

METABOLIC MAP SELECTION PROCESSING

GRAPH MAIN METABOLIC MAP — S200

CONVERT MAP INTO ADJACENCY MATRIX — S201

EXTRACT PARTIAL ADJACENCY MATRICES IN n × n (n IS THE NUMBER OF INPUT METABOLIC PATHWAY) FROM ADJACENCY MATRIX — S202

CALCULATE CORRELATION COEFFICIENTS BETWEEN PARTIAL ADJACENCY MATRIX OF INPUT METABOLIC PATHWAY AND OTHER PARTIAL ADJACENCY MATRICES — S203

SELECT METABOLIC MAP INCLUDING ADJACENCY MATRIX WHOSE ABSOLUTE VALUE OF CORRELATION COEFFICIENT IS EQUAL TO OR SMALLER THAN PREDETERMINED THRESHOLD VALUE — S204

# FIG. 13

INPUT
METABOLIC PATHWAY

PERIPHERAL COMPOUND

# FIG. 14

## ADJACENCY MATRIX

|     | ①  | ②  | 3  | ④  |
|-----|----|----|----|----|
| ①  | —  | 1  | 0  | 0  |
| ②  | 1  | —  | 1  | 1  |
| 3  | 0  | 1  | —  | 1  |
| ④  | 0  | 1  | 1  | —  |

## FIG. 15A

|   | 1 | 2 | 4 |
|---|---|---|---|
| 1 | — | 1 | 0 |
| 2 | 1 | — | 1 |
| 4 | 0 | 1 | — |

## FIG. 15B

|   | 2 | 3 | 4 |
|---|---|---|---|
| 2 | — | 1 | 1 |
| 3 | 1 | — | 1 |
| 4 | 1 | 1 | — |

## FIG. 15C

|   | 1 | 2 | 3 |
|---|---|---|---|
| 1 | — | 1 | 0 |
| 2 | 1 | — | 1 |
| 3 | 0 | 1 | — |

## FIG. 15D

|   | 1 | 3 | 4 |
|---|---|---|---|
| 1 | — | 0 | 0 |
| 3 | 0 | — | 1 |
| 4 | 0 | 1 | — |

# FIG. 16

DISTANCE MATRIX

|     | ①   | ②   | 3   | ④   |
|-----|-----|-----|-----|-----|
| ①   | 0   | 1   | 2   | 2   |
| ②   | 1   | 0   | 1   | 1   |
| 3   | 2   | 1   | 0   | 1   |
| ④   | 2   | 1   | 1   | 0   |

# FIG. 17

SYNONYMOUS EXPRESSION GENERATION PROCESSING

COLLECT COMPOUND INCLUDED IN
LITERATURE SEARCHED FOR ENZYME
IN INPUT METABOLIC PATHWAY, LABEL
COMPOUND WITH SIMILARITY TO SUBSTRATE

S300

COLLATE COMPOUND IN MAIN METABOLIC MAP
LIST WITH COMPOUND INCLUDED IN
LITERATURE SEARCHED FOR ENZYME

S301

GENERATE SYNONYM EXPRESSION LIST IN WHICH
COMPOUNDS HAVING SAME LABEL ARE COLLECTED

S302

EP 3 825 872 B1

# FIG. 18

EXAMPLE OF LABELING COMPOUND WITH SIMILARITY TO SUBSTRATE

| SIMILARITY TO SUBSTRATE | COMPOUND OF METABOLIC MAP |
|---|---|
| 0.88 | 2-acetolactate |
| 0.76 | 2-oxoisovalerate |
| 0.72 | 2-isopropylmalate |
| | |

SUBSTRATE = "2,3-dihydroxy-3-methylbutyrate"

# FIG. 19

EXAMPLE OF SIMILARITY CALCULATION

| | A (compound) | B (substrate) | Juccard COEFFICIENT |
|---|---|---|---|
| compound | 2-acetolactate | 2,3-dihydroxy-3-methylbutyrate | |
| SMILES Format | CC(=O)C(C)(O)C(O)=O | CC(C)(O)C(O)C([O-])=O | |
| Fingerprint Format | 00000000 00000000 00000000 00000000 00000000 00000000 00000000 00000000 00000100 00000000 00000000 40005000 14008000 00000000 00000000 00080008 00000000 00000000 00400000 00000001 00000000 00000a00 05000000 00000010 00000000 00000000 00000000 00000000 00000000 00000000 00000000 00000000 | 00000000 00000000 00000000 00000000 00000000 00000000 00000000 00000000 00000000 00000000 00000000 40005000 14008000 00000000 00000000 00080008 00000000 00000000 01400000 00000001 00000000 00000a00 05000000 00000010 00000000 00000000 00000000 00000000 00000000 00000000 00000000 00000000 | 0.882 |

Juccard COEFFICIENT =
$$|A \cap B| / |A \cup B|$$

34

# FIG. 20

EXAMPLE OF LITERATURE SCORE CALCULATION PROCESSING

PMID

| ID | compound | 27600064 | | 27544640 |
|---|---|---|---|---|
| 1 | 2-acetolactate | 0.8 | | 0.0 |
| | | | | |
| 65 | sulfonylurea | 0.0 | | 0.1 |
| | TOTAL | 5.8 | | 0.5 |

# FIG. 21

500

SEARCH INFORMATION OUTPUT

501    502    503

| SEARCH EXPRESSION | THE NUMBER OF SEARCHED LITERATURES | THE NUMBER OF GENE APPEARING LITERATURES |
|---|---|---|
| (2-keto-3-hydroxyisovalerate[Title/Abstract] OR 2-acetolactate[Title/Abstract] OR 3-hydroxy-3-methyl-2-oxobutyrate[Title/Abstract] OR malate[Title/Abstract] ⋮ OR l-isoleucine[Title/Abstract] ) AND (ketol-acid reductoisomerase[Title/Abstract]) | 15 | 2 |

35

# FIG. 22

600

| LITERATURE RANK | LITERATURE SCORE | LITERATURE APPEARING GENE | HOST ORGANISM | LITERATURE ID |
|:---:|:---:|:---:|:---:|:---:|
| 1 | 5.8 | ilvCD | Escherichia coli | 27600064 |
| ⋮ | ⋮ | ⋮ | ⋮ | ⋮ |
| 15 | 0.5 | – | – | 27544640 |

601   602   603   604   605

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

• JP 2002215672 A

**Non-patent literature cited in the description**

• ConTour: Data-Driven Exploration of Multi-Relational Datasets for Drug Discovery. **PARTL CHRISTIAN et al.** IEEE TRANSACTIONS ON VISUALIZATION AND COMPUTER GRAPHICS. IEEE SERVICE CENTER, 31 December 2014, vol. 20, 1883-1892